# EUROPEAN PATENT SPECIFICATION

(11) **EP 4 236 917 B1**
(45) Date of publication and mention of the grant of the patent: **04.06.2025**
(21) Application number: 22721453.3
(22) Date of filing: 11.03.2022
(51) Int. Cl.: A61K 9/127, A61K 31/704, A61P 35/00

(54) **NOVEL ANTICANCER THERAPY**
NEUE THERAPIE GEGEN KREBS
NOUVELLE THÉRAPIE ANTICANCER

(30) Priority: 11.03.2021 HU 2100104; 12.03.2021 EP 21162257
(43) Date of publication of application: 06.09.2023
(73) Proprietor: Eötvös Loránd Tudományegyetem, 1053 Budapest (HU); HUN-REN TERMÉSZETTUDOMÁNYI KUTATÓKÖZPONT, 1117 Budapest (HU)
(72) Inventor: MEZÖ , Gábor, 1087 Budapest (HU); HEGEDÜS, Kristóf, 1077 Budapest (HU); KISS, Krisztina, 1221 Budapest (HU); SZAKÁCS, Gergely, 1039 Budapest (HU); FÜREDI, András, 1024 Budapest (HU); VARGA, Zoltán, 2365 Inárcs (HU); TÓTH, Szilárd, 1101 Budapest (HU); BARTA, Gergö, 2755 Kocsér (HU); NAGYNÉ NASZÁLYI, Lívia, 1155 Budapest (HU); GAÁL, Anikó, 1117 Budapest (HU)
(74) Representative: Kompagne, Hajnalka
(86) International application number: PCT/HU2022/050023
(87) International publication number: WO 2022/189816

(56) References cited:
- US-A1- 2007 060 534
- US-B1- 6 437 105
- MEZO GABOR: "Celzott tumorterapiara alkalmas konjugatumok tervezese es szintezise", VEGYESZKONFERENCIA, 20 June 2017 (2017-06-20), XP002803851
- FORSSEN ERIC A: "The design and development of DaunoXomeB for solid tumor targeting in vivo Contents", ADVANCED DRUG DELIVERY REVIEWS FORSSEN I ADVANCED DRUG DELIVERY REVIEWS, vol. 24, no. 24, 1 January 1997 (1997-01-01), pages 133 - 150, XP055830077
- UPENDRA BULBAKE ET AL: "Liposomal Formulations in Clinical Use: An Updated Review", PHARMACEUTICS, vol. 9, no. 4, 27 March 2017 (2017-03-27), pages 12, XP055502875, DOI: 10.3390/pharmaceutics9020012
- BALLY M B ET AL: "Liposomes with entrapped doxorubicin exhibit extended blood residence times", BBA - BIOMEMBRANES ACTA, ELSEVIER, AMSTERDAM, NL, vol. 1023, no. 1, 30 March 1990 (1990-03-30), pages 133 - 139, XP023352371, ISSN: 0005-2736, [retrieved on 19900330], DOI: 10.1016/0005-2736(90)90018-J
- BOLOTIN E M ET AL: "AMMONIUM SULFATE GRADIENTS FOR EFFICIENT AND STABLE REMOTE LOADING OF AMPHIPATHIC WEAK BASES INTO LIPOSOMES AND LIGANDOLIPOSOMES", JOURNAL OF LIPOSOME RESEARCH, TAYLOR & FRANCIS, PHILADELPHIA, PA, US, vol. 4, no. 1, 1 January 1994 (1994-01-01), pages 455 - 479, XP000572717, ISSN: 0898-2104

## Description

### FIELD OF THE INVENTION

The invention relates to the field of cancer therapy. In particular, a highly effective chemotherapy is provided in the form of liposome-encapsulated 2-deamino-2-pyrrolino-daunorubicin, which has been found to eliminate cancer cells and increase overall survival. Furthermore, treatment with liposome-encapsulated 2-deamino-2-pyrrolino-daunorubicin caused no severe undesired effects in mouse models of different cancer types.

### BACKGROUND OF THE INVENTION

Doxorubicin (Dox) and daunorubicin (Dau), also known as daunomycin, are anthracycline anti-cancer agents; Dox is used in the treatment of breast cancer, acute leukemia, bladder cancer, etc., while Dau is used predominantly in the treatment of acute and chronic leukemia. US6437105 and US2007/060534 describe the synthesis of other anthracycline analogues and suggest their use in the treatment of cancer. Nagy et al. (Cytotoxic analogs of luteinizing hormone-releasing hormone containing doxorubicin or 2-pyrrolinodoxorubicin, a derivative 500-1000 times more potent. Proc. Natl. Acad. Sci. USA Vol. 93, pp. 7269-7273, July 1996 Medical Sciences and Synthesis and biological evaluation of cytotoxic analogs of somatostatin containing doxorubicin or its intensely potent derivative, 2-pyrrolinodoxorubicin. Proc. Natl. Acad. Sci. USA Vol. 95, pp. 1794-1799, February 1998 Medical Sciences) described the synthesis and cytotoxicity of a derivative of Dox, 2-pyrrolino-doxorubicin (PyDox), which appeared 500-1000 times more potent in vitro than Dox. Mezö (Mezö Gábor: Célzott tumorterápiára alkalmas konjugátumok tervezése és szintézise, Vegyészkonferencia 2017. 06. 20., Hajdúszoboszló) reported an IC₅₀ of 0,0002 ±0,0001 µM on MCF-7 human breast adenocarcinoma cells and HT-29 human colon adenocarcinoma cells, of 2-deamino-2-pyrrolino-daunorubicin (PyDau), which is three orders of magnitude lower than the IC₅₀ of daunorubicin on the same cells

US2007/060534 does not disclose any biological data regarding 2-deamino-2-pyrrolino-daunorubicin and US6437105 only describes data similar to the data in Mezö (supra). Examinations proved that the 2-pyrrolino modification of both Dox and Dau resulted in overly toxic compounds that are unsuitable for therapeutic use. Jungwirth et al. (Regression of rat Dunning R-3327-H prostate carcinoma by treatment with targeted cytotoxic analog of luteinizing hormone releasing hormone AN-207 containing 2-pyrrolinodoxorubicin. International Journal of Oncology 10: 877-884, 1997) showed in a rat Dunning R-3327-H prostate carcinoma model that administering a dose of 50 nmol/kg of PyDox resulted in the death of all rats and 6 of 10 animals in the group that received 3X25 nmol/kg PyDox died. Jungwirth et al. also found no significant change in the number of mitotic or apoptotic cells after PyDox treatment in rats. Although Nagy et al. (Design, synthesis, and in vitro evaluation of cytotoxic analogs of bombesin-like peptides containing doxorubicin or its intensely potent derivative, 2-pyrrolinodoxorubicin. Proc. Natl. Acad. Sci. USA Vol. 94, pp. 652-656, January 1997 Medical Sciences) suggested that "2-pyrrolino-DOX and its cytotoxic BN conjugates could be used in preference to DOX or its analogs for the treatment of cancers", they also reported that "16 of 18 hamsters died after intraperitoneal administration of a total dose of 100 nmol/kg of" PyDox in a study of nitrosamine-induced pancreatic cancers in golden hamsters.

Similarly, PyDau appeared toxic or even lethal below doses that are required for an anticancer effect. Dose-finding studies in mice also indicated that the safety window of PyDau is too narrow to use the compound as an anticancer treatment. Due to their increased toxicity, the pyrrolino derivatives of neither doxorubicin nor daunorubicin were considered as promising drug candidates on their own.

Encapsulating a cytotoxic drug into a liposome is a known method to ameliorate its undesired effects (non-specific toxicity), presumably due to the sustained release of the liposomal drug (Bally et al. (1990) Liposomes with entrapped doxorubicin exhibit extended blood residence times. Biochim. Biophys. Acta. 1023, 133-139.). Liposome-encapsulated doxorubicin (Dox), a structural relative of daunorubicin has a more acceptable side-effect profile than non-liposomal doxorubicin and is widely used in cancer therapy.

Not yet published data of the present inventors show that liposome encapsulation did not significantly affect the toxicity of PyDau *in vitro.* Bolotin et al. (Ammonium Sulfate Gradients For Efficient And Stable Remote Loading Of Amphipathic Weak Bases Into Liposomes And Ligandoliposomes. Journal of Liposome Research, 4(1), 455-479 (1994)) showed that the amino group of Dox, which is modified in PyDau (and PyDox), plays an important role in the formation of gel-like precipitates (liquid crystal structure) inside the liposomes. The liquid crystal structure is thought to ensure the sustained release of the active from the liposome. Therefore, research efforts have focused on targeting PyDox and PyDau through peptide conjugation (Nagy et al. Proc. Natl. Acad. Sci. USA Vol. 94, pp. 652-656, January 1997, supra; Mezö supra; Castex et al. 2-Pyrrolinodoxorubicin and its peptidevectorized form bypass multidrug resistance. Anti-Cancer Drugs 2004, 15:609-617.) rather than encapsulating these compounds in a liposome. Indeed, not yet published data of the present inventors suggest that PyDau, in sharp contrast with Dox, shows no liquid crystal structure inside a liposome.

The need for potent, safe and possibly curative antitumor compounds and formulations still exists.

### SUMMARY OF THE INVENTION

Here we report that a liposome-encapsulated form of 2-deamino-2-pyrrolino-daunorubicin may be used for effectively killing cancer cells when administered *in vivo,* in spite of the fact that *in vitro* toxicity studies showed that liposomal encapsulation did not decrease the toxicity of PyDau. Results of the *in vitro* toxicity studies were further supported by the structural characterization of LiPyDau samples and comparison thereof with liposomal doxorubicin (LiDox) and liposomal pyrrolino-doxorubicin (LiPyDox) samples: the liquid crystalline phase of encapsulated drug characteristic of liposomal doxorubicin is not observable for liposomal PyDau and liposomal PyDox.

Intriguingly, LiPyDau was proven a safe and surprisingly effective anticancer therapy in several animal models. Whereas the applicability of PyDau was prevented by its high systemic toxicity, treatment with the liposomal form thereof increased the overall survival of tumor-bearing mice significantly, was able to cure cancer in the model animals and proved to be effective even in tumors resistant to pegylated liposomal doxorubicin (PLD).

The invention provides liposome-encapsulated 2-deamino-2-pyrrolino-daunorubicin for use in a method of treating a disease.

In a preferred embodiment the disease is cancer, preferably a solid tumor, preferably breast cancer, lung cancer, melanoma, Kaposi's sarcoma, or neuroblastoma.

Preferably, the cancer is a drug-naïve cancer, i.e., a cancer that has not been treated with a chemotherapeutic agent prior to the treatment with LiPyDau.

**In** another preferred embodiment the cancer is therapy resistant (refractory) cancer, and the patient having the refractory cancer has been administered a chemotherapeutic agent other than LiPyDau or a combination of chemotherapeutic agents, the combination not comprising LiPyDau prior to the administration of LiPyDau.

**In** a preferred embodiment the therapy resistant cancer is resistant to treatment with an anthracycline compound, preferably to doxorubicin. **In** another preferred embodiment the therapy resistant cancer is resistant to treatment with a liposomal form of doxorubicin, preferably pegylated liposomal doxorubicin. **In** another preferred embodiment the therapy resistant cancer is resistant to both a non-liposomal form of doxorubicin and to a liposomal form of doxorubicin.

Preferably the cancer is a P-glycoprotein (Pgp) overexpressing cancer.

Preferably the cancer is selected from the group consisting of breast cancer, uterine cancer, lung cancer, melanoma and cancers resistant to liposomal doxorubicin treatment and multidrug resistant cancers. Preferably the cancer is triple-negative breast cancer. Preferably the cancer is lung carcinoma, preferably lung adenocarcinoma. Preferably the cancer is metastatic melanoma.

In preferred embodiments the cancer is recurrent cancer. In preferred embodiments the patient having recurrent cancer has been administered a chemotherapeutic agent other than LiPyDau, preferably with an anthracycline compound, more preferably with doxorubicin or a combination of chemotherapeutic agents (preferably comprising an anthracycline compound, more preferably doxorubicin), the combination not comprising LiPyDau prior to the recurrence of the cancer.

Preferably the treatment is curative treatment, i.e., the cancer cells cannot be detected after cessation of the treatment. The treatment may be preventive treatment.

Preferably the liposome comprises:
- cholesterol, fully hydrogenated soy phosphatidylcholine (HSPC), N-(carbonyl-methoxypolyethylene glycol 2000)-1,2-distearoyl-sn-glycero-3-phosphoethanolamine sodium salt (MPEG-DSPE);
- HSPC, 1,2-distearoyl-sn-glycero-3-phosphoethanolamine, cholesterol;
- cholesterol, phosphatidylcholine, citric acid, NaOH or
- cholesterol and distearoylphosphatidylcholine.

**In** a preferred embodiment the liposome consists of
- cholesterol, fully hydrogenated soy phosphatidylcholine (HSPC), N-(carbonyl-methoxypolyethylene glycol 2000)-1,2-distearoyl-sn-glycero-3-phosphoethanolamine sodium salt (MPEG-DSPE);
- HSPC, 1,2-distearoyl-sn-glycero-3-phosphoethanolamine, cholesterol;
- cholesterol, phosphatidylcholine, citric acid, NaOH or
- cholesterol and distearoylphosphatidylcholine.

The invention further provides a pharmaceutical composition comprising liposome-encapsulated 2-deamino-2-pirrolino-daunorubicin and a pharmaceutically acceptable excipient.

### BRIEF DESCRIPTION OF THE FIGURES

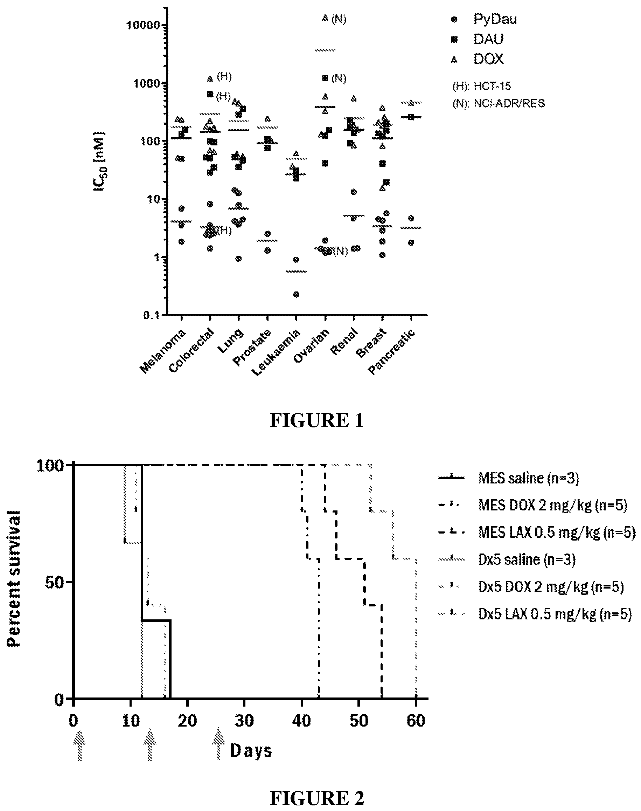

**Figure 1****.** In vitro toxicity of PyDau (dots), in comparison to daunorubicin (rectangles, Dau) or doxorubicin (triangles, DOX). Data points represent average IC50 values of at least 3 replicates, measured in different cell lines grouped according to the tissue of origin. Horizontal lines indicate average IC50 values within the groups. H and N denote multidrug resistant cells expressing P-glycoprotein (H: HCT-15; N: NCI-ADR/RES).
**Figure 2****.** LiPyDau is superior to DOX in treating parental MES-SA (MES) and multidrug resistant, Pgp overexpressing MES-SA/Dx5 (Dx5) xenografts. Treatments at the same time with either saline, DOX (2 mg/kg iv.), or LiPyDau (LAX, 0.5 mg/kg iv.) are indicated by arrows.

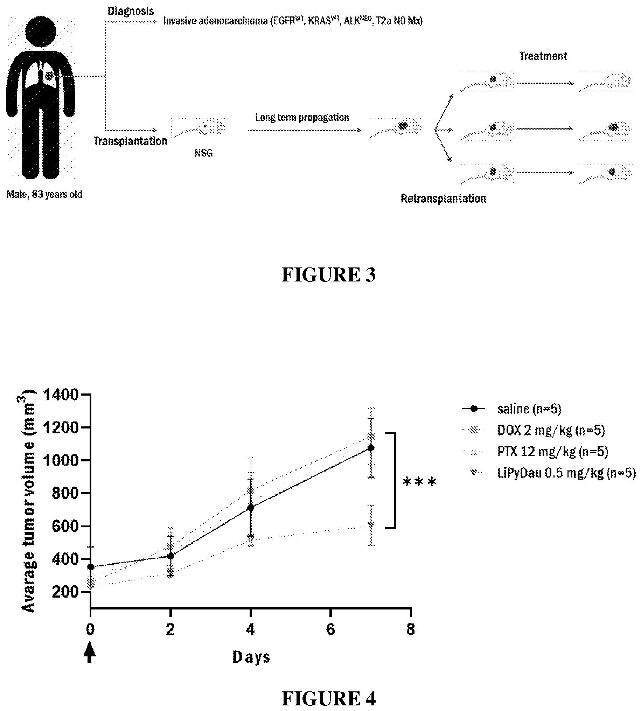

**Figure 3****.** Strategy for the establishment of the patient derived xenograft (PDX) model.
**Figure 4****.** LiPyDau treatment via tail vein injection inhibits the growth of patient-derived lung adenocarcinoma xenografts in NOD scid gamma (NSG) mice after 7 days. Tumor growth of a lung adenocarcinoma PDX model treated with saline, DOX (2 mg/kg), paclitaxel (PTX; 12 mg/kg)), or LiPyDau (0.5 mg/kg). The time of treatment is indicated by the arrow. Data represent the mean ± SEM (***P <0.001). Statistical analysis was made with two-way ANOVA and Tukey's multiple comparison test.

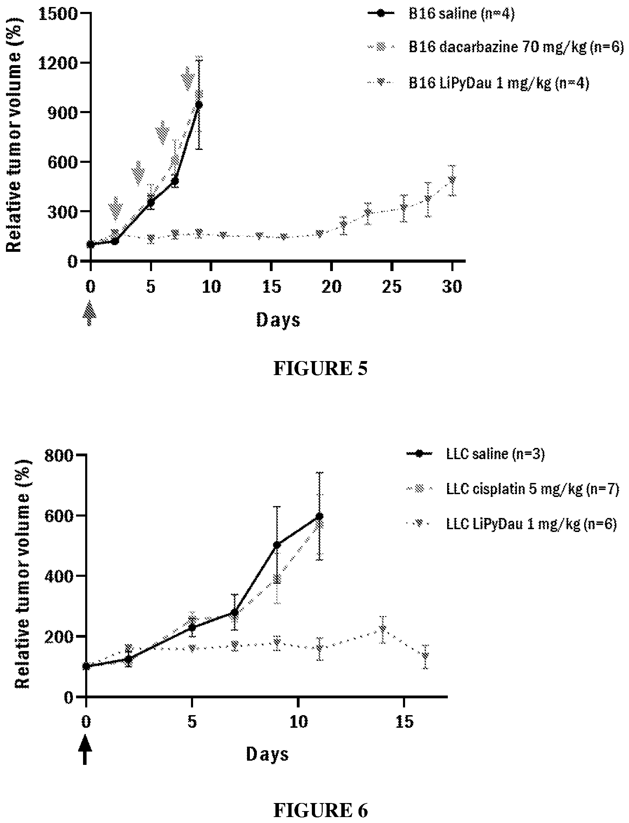

**Figure 5****.** Anti-tumor effect of LiPyDau and dacarbazine in the B16 mouse melanoma model. 5×10⁵ B16 melanoma cells in 100 µl PBS were subcutaneously inoculated into the right flank of C57BL/6 mice. Mice were randomly grouped to receive control treatment (saline, filled circles), dacarbazine (filled squares; 70 mg/kg), or LiPyDau (filled triangles; 1 mg/kg)) through tail vein injection. Downward arrows indicate repeated administrations of dacarbazine and saline, while the upward arrow indicates a single dose of LiPyDau.
**Figure 6****.** Anti-tumor effect of LiPyDau and cisplatin in the mouse Lewis lung cancer (LLC) lung carcinoma model. C57BL/6 mice received sc. injection of 2×10⁵ LLC cells in 100 µl PBS. When the tumors were palpable, mice were randomized to receive a single intravenous dose of cisplatin (5 mg/kg) or LiPyDau (1 mg/kg) as indicated by the arrow. Control mice were treated with saline.

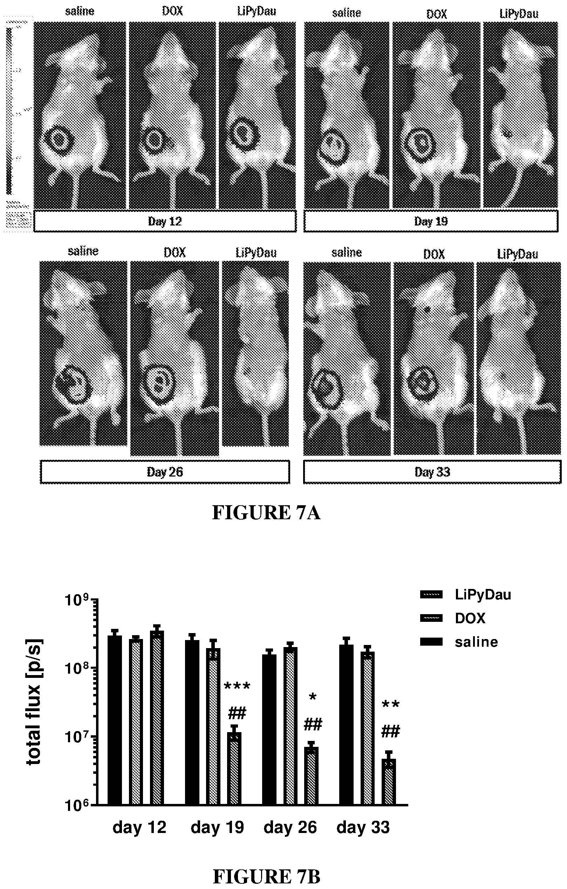

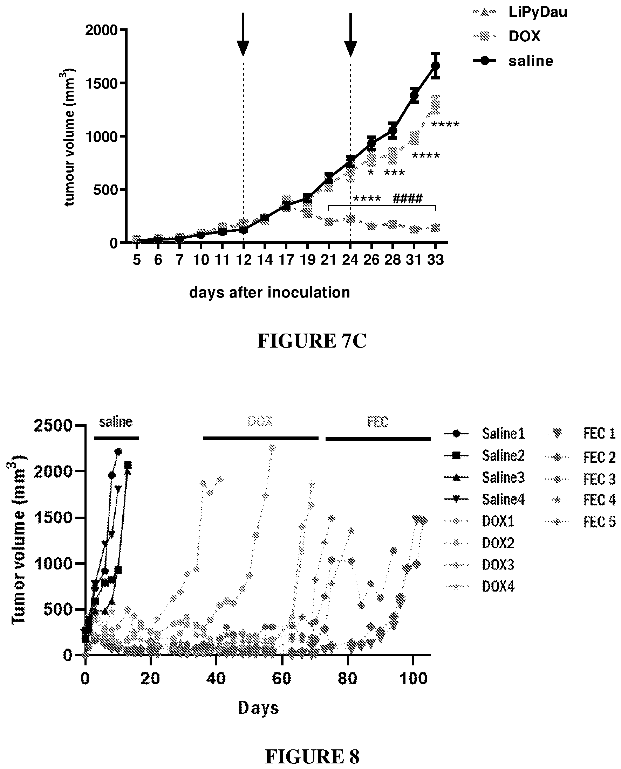

**Figure 7****.** Evaluation of the activity of 1 mg/kg LiPyDau, 5 mg/kg doxorubicin (DOX) and saline in the murine 4T1-LUC mammary tumor model. A. Bioluminescence imaging and monitoring of tumor growth in the same mice over 21 days. B. Quantification of total photon flux (the number of photons/second, p/s) at tumor regions shown in A. Statistical analysis was made with two-way ANOVA and Tukey's multiple comparison test (* p<0.05, ** p<0.01, *** p<0.001 vs. saline; ## p<0.01 vs. DOX). C. Effect of treatment on tumor volumes, as determined by caliper measurements. Arrows indicate treatment. Statistical analysis was made with two-way ANOVA and Tukey's multiple comparison test (* p<0.05, **** p<0.0001 vs. saline; #### p<0.0001 vs. DOX).
**Figure 8****.** Growth kinetics of individual Brca1^{-/-};p53^{-/-} tumors engrafted into the mammary fat pad of wild-type female FVB mice. Treatment with saline, 5 mg/kg DOX, or combination of 24 mg/kg Fluoruracil, 4 mg/kg Epirubicin and 120 mg/kg Cyclophosphamide (FEC).

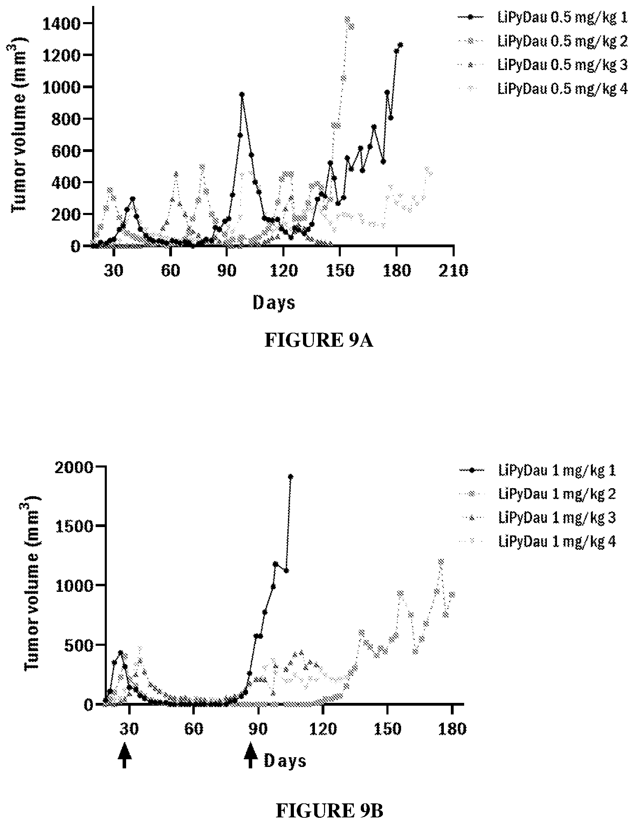

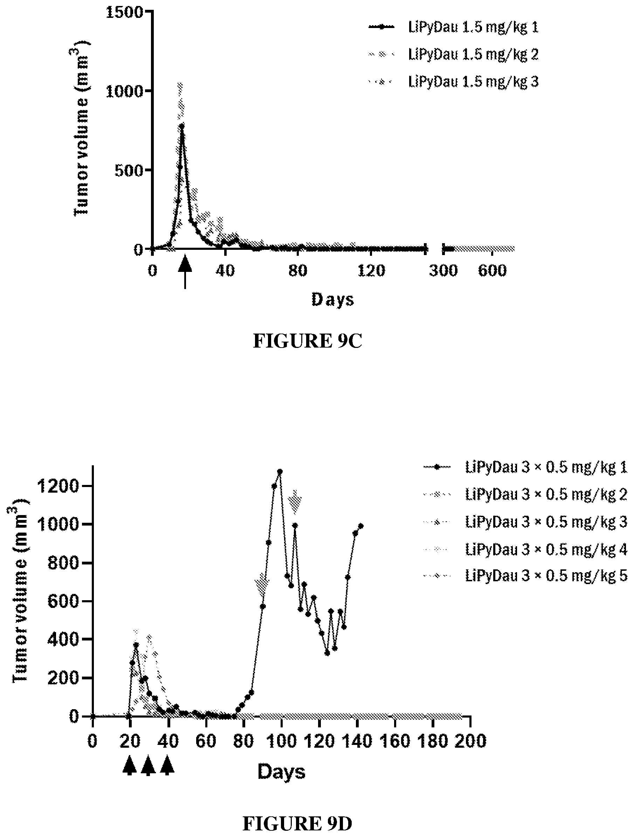

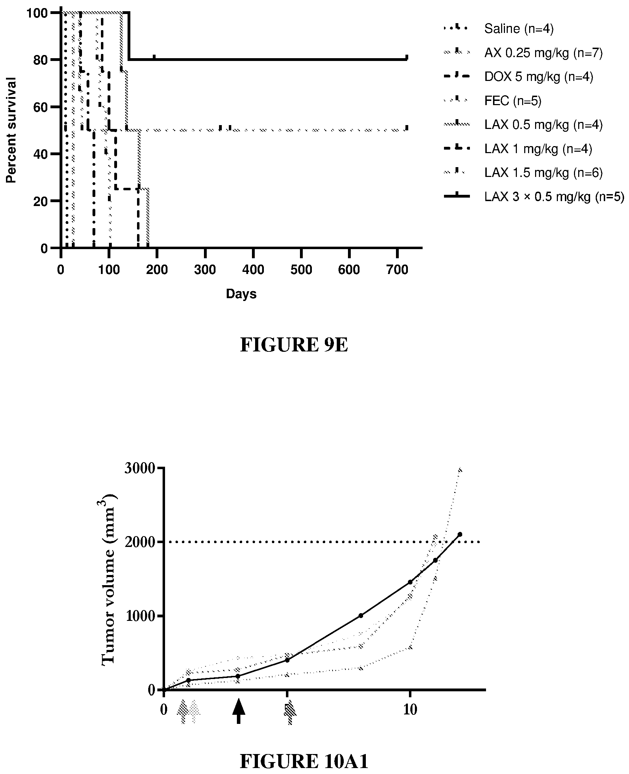

**Figure 9****.** Growth kinetics of individual Brca1^{-/-};p53^{-/-} tumors engrafted into the mammary fat pad of wild-type female FVB mice. Mice were treated with 0.5 mg/kg (A), 1 mg/kg (B) or 1.5 mg/kg (C) LiPyDau. LiPyDau was also administered in three 0.5 mg/kg doses, distributed over 21 days (0.5 mg/kg/week, D). Overall survival of each group compared to previously presented treatments (PyDau, DOX, FEC) is shown in E.

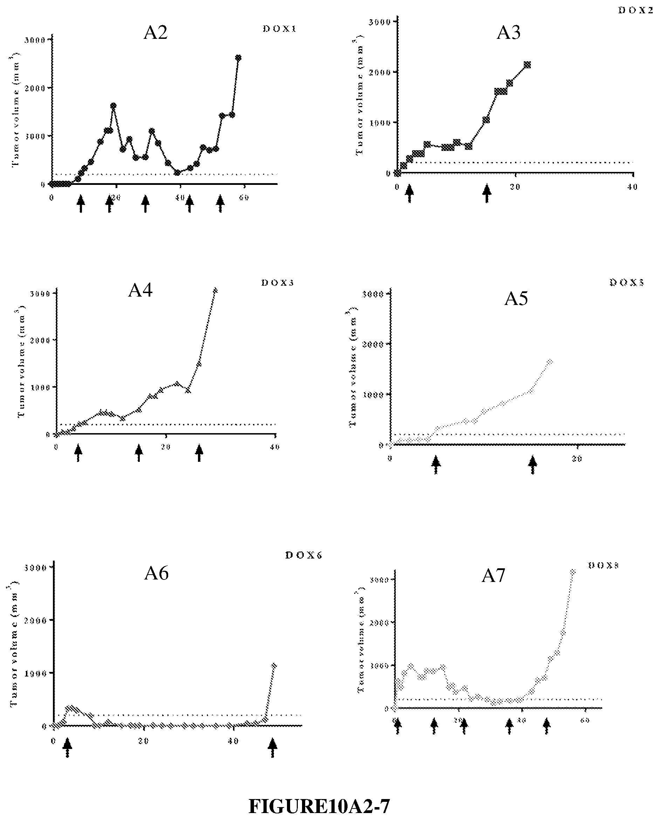

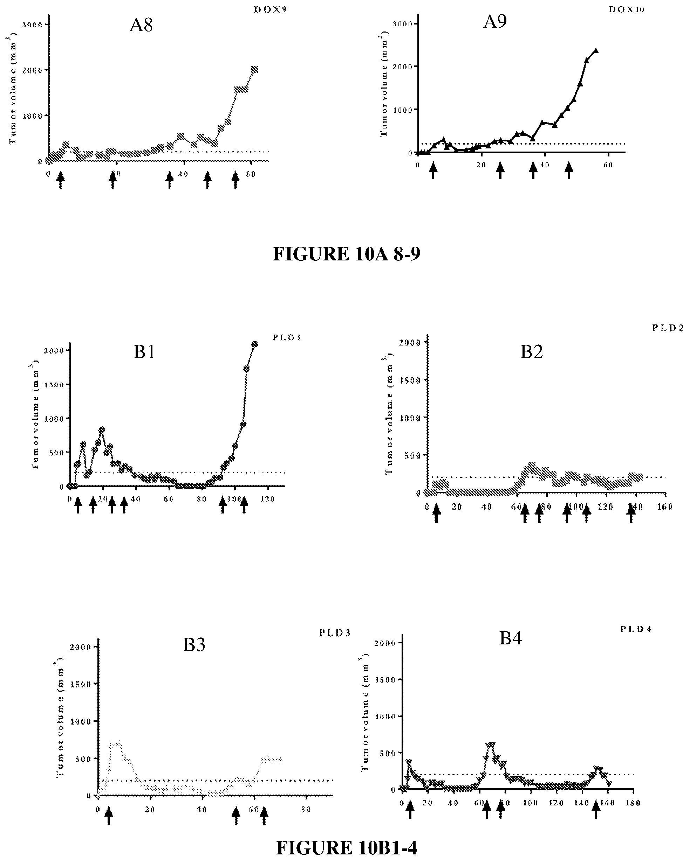

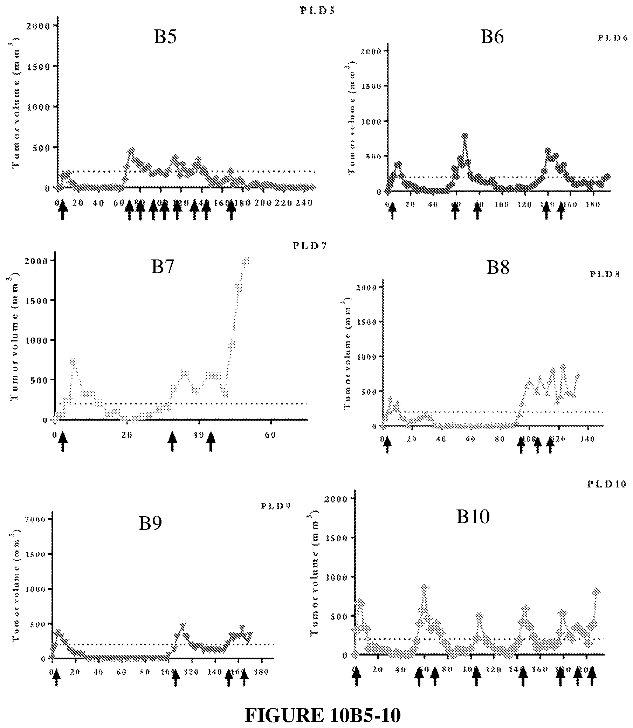

**Figure 10****.** Comparison of the antitumor activity of DOX, pegylated liposome encapsulated DOX (PLD), PyDau and LiPyDau in the same model as in Figure 8 and Figure 9. A. 10A1 shows treatment with saline. Treatment of individual tumors (10A2-9; DOX1-10) with Maximum Tolerable Dose or MTD (5 mg/kg) DOX is indicated by the arrows. B. Treatment of individual tumors (10B1-B10; PLD1-10) with MTD (8 mg/kg) PLD. (X axis: number of days; Figure from Füredi et al. supra)

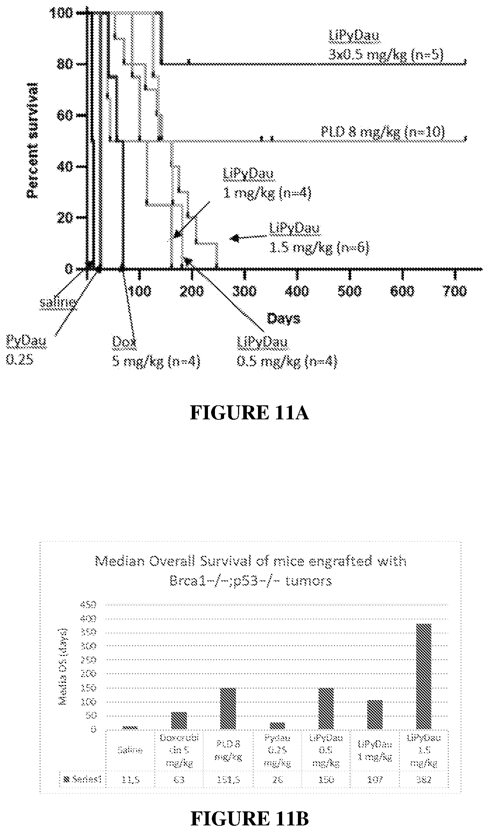

**Figure 11****.** A. Overall survival of mice in the same model as in Figure 8, 9 and Figure 10. Mice were treated with the MTD of DOX (blue) or PLD (orange), PyDau (purple) or various doses of LiPyDau. B. Median overall survival of the same treatment groups.

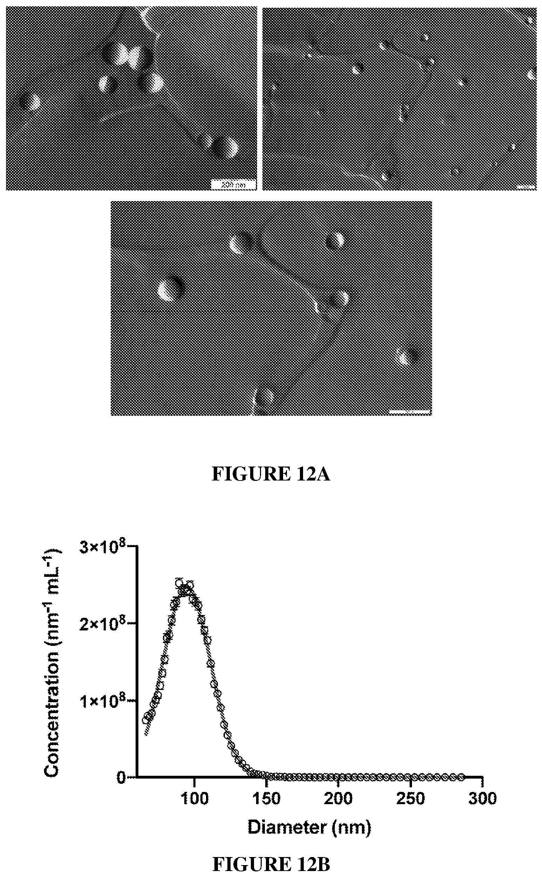

**Figure 12****.** A) FF-TEM image of PyDau loaded liposomes. (B) Size distribution of PyDau liposomes determined by microfluidic resistive pulse sensing. Symbols represent measured data points for each size bin; the solid line corresponds to the fitted Gaussian function. (C) FF-TEM images of liposomal PyDox.

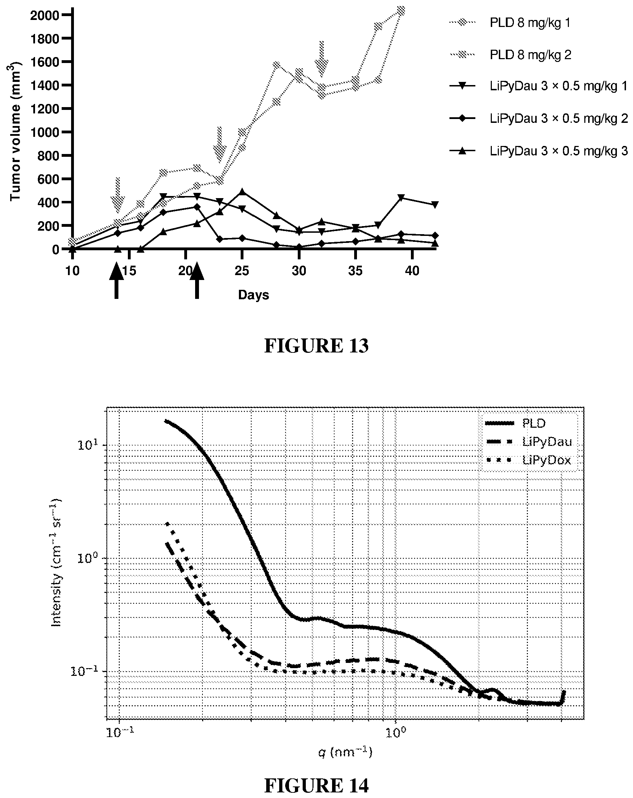

**Figure 13****.** Growth kinetics of PLD-resistant Brca1^{-/-};p53^{-/-} tumors engrafted into the mammary fat pad of wild-type female FVB mice. Mice were treated with 3 × 0.5 mg/kg LiPyDau or 8 mg/kg PLD. To reduce toxic side-effects, LiPyDau was administered in three 0.5 mg/kg doses, distributed over 21 days (0.5 mg/kg/week). Grey arrows show PLD treatments, while black arrows indicate the start of LiPyDau therapy. The first black arrow marks the first dose of LiPyDau for mouse 1 and 2 and the second black arrow shows the start of treatment for mouse 3.
**Figure 14****.** Small-angle X-ray scattering curves of Caelyx-, liposomal PyDau and liposomal PyDox samples.

### DETAILED DESCRIPTION OF THE INVENTION

The invention provides liposome-encapsulated 2-deamino-2-pyrrolino-daunorubicin, for use in a method for treating cancer. Treatment with LiPyDau may be useful even in the case of recurring and/or treatment-refractory cancer. LiPyDau may also be used effectively in cases where the cancer has already been treated with other unencapsulated, liposome encapsulated or otherwise formulated anthracycline derivatives (such as doxorubicin) and/or other chemotherapeutic agents before recurrence. Resistance mediated by Pgp does not develop against LiPyDau, therefore it may be used repeatedly. Surprisingly, LiPyDau treatment resulted in a remarkable number of total recoveries from cancer in animals, strongly suggesting that this treatment may be curative in humans as well.

### Chemical structure of 2-deamino-2-pirrolino-daunorubicin:

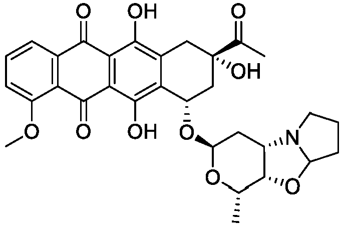

The term "preventing" or "prevent" refers to (a) keeping a disorder from occurring or (b) delaying the onset of a disorder or onset of symptoms of a disorder.

The term "treating," "treatment," or "treat" refers to abrogating a disorder, reducing the severity of a disorder, or reducing the severity or occurrence frequency of a symptom of a disorder.

The term "curative" or "curing" refers to (the goal of) achieving a complete remission and preventing the recurrence of cancer.

Pharmaceutically acceptable liposomes are well known in the art and may be used to obtain LiPyDau for use according to the invention. For a guidance on liposomes see e.g. Immordino et al. Stealth liposomes: review of the basic science, rationale, and clinical applications, existing and potential. Int J Nanomedicine. 2006 Sep; 1(3): 297-315; Bulbake et al. Liposomal Formulations in Clinical Use: An Updated Review. Pharmaceutics. 2017 Mar 27;9(2):12.

Dose-finding studies in mice indicated that the safety window of PyDau is too narrow to use the compound as an anti-cancer treatment. *In vivo* mice studies showed that liposome encapsulation of PyDau drastically reduced the adverse toxicity thereof while LiPyDau still destroyed cancer cells. LiPyDau appears to be a safe and effective therapy against different types of cancer (table 1 and 2).

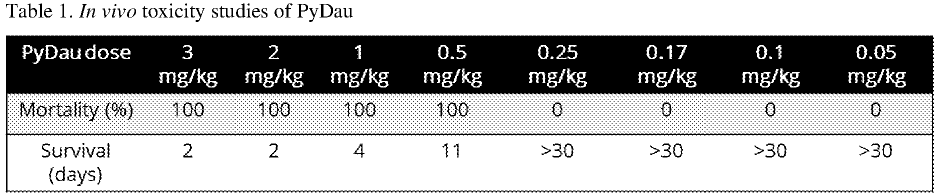

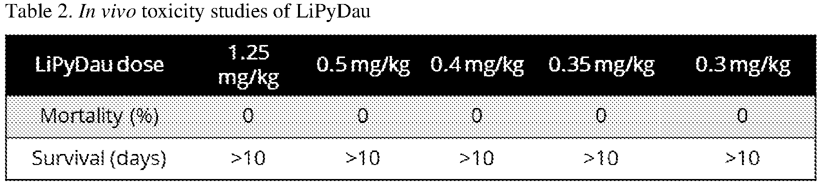

Furthermore, unlike most anthracycline derivatives, the toxicity of PyDau is not diminished by Pgp, and therefore PyDau overcomes multidrug resistance. LiPyDau retains this trait.

### Significant in vitro toxicity of PyDau across a panel of cancer cell lines of diverse origin

The cytotoxicity of PyDau was tested against a panel of cell lines of various tissue of origin (Figure 1). PyDau was toxic to every cell line with IC₅₀ values ranging from 0.23 nM to 14.3 nM, typically between 1-10 nM. Daunorubicin and DOX were less toxic, IC₅₀ values were typically between 30-300 nM. PyDau was effective also against the Pgp expressing multidrug resistant HCT-15 and NCI/ADR-RES cells.

### In vitro toxicity of PyDau

Liposome encapsulation of Dox remarkably decreases its toxicity, making it safer and opening its therapeutic window considerably (Tables 3A and B).

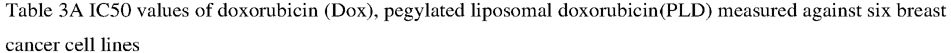

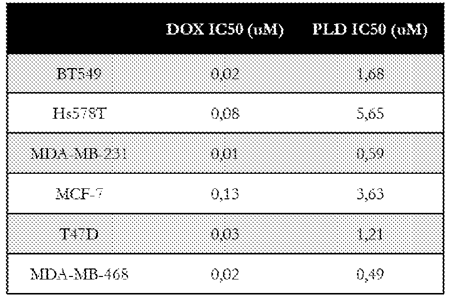

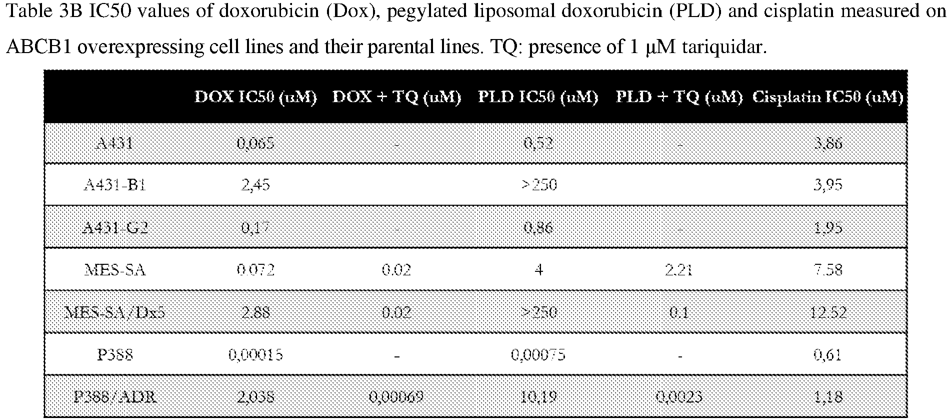

In contrast, liposome encapsulation did not significantly affect the toxicity of PyDau (Table 4).

Table 4 IC50 values of PyDau, pegylated liposomal PyDau (LiPyDau) against cancer cell lines. TQ: presence of 0.4 µM tariquidar.

| | PyDau [nM] | PyDau+TQ [nM] | LiPyDau [nM] | LiPyDau+TQ [nM] |
|---|---|---|---|---|
| Malme-3M | 1.84 | | 1.63 | |
| SK-MEL-28 | 6.87 | | 6.21 | |
| HT29 | 3.05 | | 1.83 | |
| HCT-15 | 2.86 | | 1.56 | |
| Mes-Sa | 1.35 | 1.63 | 1.71 | 1.80 |
| Mes-Sa/Dx5 | 2.51 | 1.65 | 4.29 | 1.98 |

### Broad antitumor activity of LiPyDau in diverse mouse models of cancer

To evaluate therapeutic potential, the anticancer activity of LiPyDau was assessed in diverse mouse models of cancer. LiPyDau exhibited a broad anticancer activity across allograft and xenograft models, including cancers showing resistance to clinically used chemotherapeutics. Treatment with LiPyDau was curative in a genetically engineered mouse model of triple-negative breast cancer. No significant change in body weight associated with LiPyDau treatment was observed compared with control-treated animals.

### Xenograft tumors in mice

The antitumor effect of LiPyDau was evaluated on the in vivo tumor growth of MES-SA and MES-SA/Dx5 xenografts (Figure 2). As expected, doxorubicin treatment was largely ineffective in mice engrafted with the multidrug resistant MES-SA/Dx5 cells, whereas the same treatment increased the overall survival of mice bearing MES-SA derived tumors. In contrast, DOX-resistant MES-SA/Dx5 tumors responded to LiPyDau treatment, resulting in a significant prolongation of the overall survival of mice.

### Human patient-derived tumors in mice

A Patient-derived xenograft (PDX) model was developed to assess the efficacy of LiPyDau treatment (Figure 3). Briefly, tumor pieces derived from an 83-year-old human patient with invasive lung adenocarcinoma were engrafted in mice subcutaneously. LiPyDau significantly suppressed the growth of patient-derived xenograft adenocarcinoma tumors, while DOX and paclitaxel failed to exert any effect on tumor growth (Figure 4).

### Allograft tumor models

The antitumor activity was also evaluated in mouse allograft models. The efficacy of LiPyDau was compared to the antitumor potential of clinically used chemotherapies including dacarbazine and cisplatin. LiPyDau exhibited significant and long-lasting anticancer activity across all models.

### Mouse B16 melanoma model

As shown in Figure 5, repeated treatments with dacarbazine, the only single-agent approved by the Food and Drug Administration for treating metastatic melanoma, did not exhibit any effect on the growth of B16 melanoma tumors compared to the control group treated with saline. In contrast, treatment with a single dose of LiPyDau resulted in an almost complete inhibition of tumor growth for at least 20 days, without causing a loss of body weight.

### Mouse Lewis Lung Carcinoma (LLC) model

The murine Lewis Lung Carcinoma (LLC) tumor model is a syngeneic lung cancer model widely used for the evaluation of the efficacy of chemotherapeutic agents in vivo. Treatment with cisplatin did not have any measurable effect on tumor growth, as compared to control (saline). In contrast, a single dose of LiPyDau resulted in a significant antitumor effect (Figure 6).

### Mouse 4T1 breast cancer model

Inoculation of luciferase-expressing 4T1 (4T1-LUC) cells in the mammary fat pad of female mice results in the formation of a highly malignant and metastatic tumor, offering a syngeneic and orthotopic breast tumor model. Expression of the luciferase allows the monitoring of the location, growth and viability of tumor cells *in vivo* by bioluminescence. As shown on Figure 7, DOX had a small but significant effect on the growth of tumors despite no detectable differences in *in vivo* viability of cancer cells according to bioluminescence imaging of luciferase activity. While DOX-treated mice displayed a tumor progression similar to the control, LiPyDau could suppress the tumor growth, resulting in the almost complete elimination of tumor cells and a prolonged antitumor effect up to the experimental endpoint (day 33).

### Genetically engineered mouse model of triple-negative breast cancer

The therapeutic value of LiPyDau was also tested in a clinically relevant murine model of hereditary triple negative breast cancer. Wild-type FVB mice were orthotopically transplanted with mammary tumor pieces obtained from Brca1^{-/-};p53^{-/-} mice. This model closely mimics cancer in human patients and therefore offers a unique opportunity to study response to treatment. Like most human cancers, Brca1^{-/-};p53^{-/-} tumors show initial sensitivity to doxorubicin and combination treatments such as FEC (5-fluorouracil, epirubicin and cyclophosphamide), but the tumors always acquire resistance (Figure 8).

Treatment with LiPyDau resulted in a marked, dose-dependent antitumor response, curing several mice (Figure 9).

Next, treatment with DOX, PLD and LiPyDau was compared in the same model. Figure 10 shows that all tumors developed resistance to treatment with Maximum Tolerable Dose or MTD (5 mg/kg) DOX (A) and that the increased efficacy of PLD (treatment with the MTD, 8 mg/kg) is due to the delayed onset of resistance against PLD. Despite increased median overall survival (OS), none of the mice are cured by PLD. 2 tumors became resistant to PLD. Mice with responding tumors had to be sacrificed due to accumulated toxicity.

### LiPyDau inhibits the growth of PLD-resistant tumors in vivo

It was shown in the Brca1^{-/-};p53^{-/-} tumor model that, while PLD treatment was significantly more efficient than DOX, it still couldn't cure the mice and the relapsing tumors expressed high levels of Pgp, sometimes 100-fold higher than DOX-resistant tumors (Füredi et al, Journal of Controlled Release, 2017). Remarkably, 3 repeated doses of 0.5 mg/kg LiPyDau treatment successfully inhibited the growth of PLD-resistant tumors. In contrast, PLD treatment proved to be ineffective and the developed drug resistance prevented remission, tumors reached the 2000mm³ limit in 30 days (Figure 11).

Of note, PLD also resulted in an increase of median overall survival of Brca1^{-/-};p53^{-/-} tumor-engrafted mice, due to the delayed onset of resistance against PLD. Despite repeated treatments with PLD, mice were not cured and resistance developed eventually.

### Preparation of PyDau

A method for the preparation of 2-pyrrolino-daunorubicin is also provided, comprising
reacting daunorubicin hydrochloride with 4-bromobutyraldehyde to convert daunorubicin hydrochloride into 2-pyrrolinodaunorubicin.

In a preferred embodiment daunorubicin hydrochloride is added to a solution of N,N-diisopropylethylamine in dichloromethane prior the reaction with 4-bromobutyraldehyde.

4-bromobutyraldehyde may be prepared by reacting tetrahydrofuran with HBr to obtain 4-bromobutanan-1-ol, which is then reacted with pyridinium chlorochromate in dichloromethane to obtain 4-bromobutyraldehyde, according to Mezö Gábor: Célzott tumorterápiára alkalmas konjugátumok tervezése és szintézise, Vegyészkonferencia 2017. 06. 20., Hajdúszoboszló:

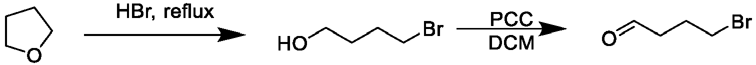

*Mode of administration:* LiPyDau or the pharmaceutical composition comprising LiPyDau is preferably administered as an intravenous infusion.

*Prodrugs.* Instead of LiPyDau, prodrugs of PyDau (i.e. compounds which upon administration to a patient are converted into PyDau) may be used in any one of the aspects and embodiments of the present invention, either in liposomal form or in a non-liposomal formulation, where it is appropriate or desired.

*Conjugated LiPyDau and conjugated prodrugs of PyDau.* Instead of LiPyDau or a prodrug of PyDau it may be preferable to use conjugated forms thereof. Conjugation with a targeting moiety, such as [D-Lys6]-GnRH-I results in a better targeting of the cancer cells and thus increased effectivity and decreased side-effects.

### EXAMPLES

### Novel improved synthetic approach for the development of 2-pyrrolino-anthracycline derivatives

2-pyrrolinodoxorubicin (PyDox) TFA salt was developed in 1996 (Nagy A. et al.; Proc. Natl. Acad. Sci. U.S.A. 93 (1996), 2464-2469). In the first step Nagy et al. applied 4-iodobutyraldehyde, that was prepared from 2-(3-chloropropyl)-1,3-dioxolane followed by Shiff-base formation and cyclization. The synthesis of 4-iodobutyraldehyde - which is the key step for efficient synthesis - was later improved by Studenovsky et al. (Eur. J. Pharm. Sci. 42 (2011), 156-163). However, this procedure resulted in a significant amount of side product corresponding to the dimer derivative of PyDau, most probably connected through the pyrrolino moiety, Therefore, a new approach was investigated for the preparation of PyDau. First, the stable and storable 4-bromobutyraldehyde was applied for the synthesis. The reaction was carried out in DCM in the presence of excess DIPEA under nitrogen atmosphere at room temperature (RT) for 10 h. This reaction allowed an easier workup procedure that resulted in the monomer form of PyDau in a high content (80% purity) that was stable for long time at -20 °C. Using this new synthetic method the synthesis of 4-iodobutyraldehyde could be eliminated and the formation of side products could be decreased significantly in the preparation of 2-pyrrolino-anthracycline derivatives in large scale.

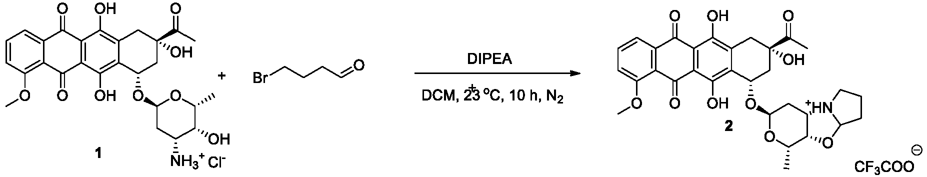

### Synthesis of 2-pyrrolinodaunorubicin

To a solution of *N,N*-diisopropylethylamine (1.00 g, 7.74 mmol, 4.35 equiv) in dichloromethane (100 mL) was added daunorubicin hydrochloride 1 (1.00 g, 1.77 mmol, 1 equiv) at 23 °C under nitrogen atmosphere. The suspension was stirred vigorously until the hydrochloride salt was completely dissolved. The resulting deep purple solution was stirred for 15 minutes at the same temperature and 4-bromobutyraldehyde (0.54 g, 3.5 mmol, 2.0 equiv) was added in one portion. The reaction mixture was stirred until high-performance liquid chromatography analysis indicated full conversion of daunorubicin (6-10 h). The reaction mixture was concentrated under reduced pressure (20-25 mL) and the resulting deep red oil was diluted with diethyl ether (100 mL). The resulting suspension was stirred for 10 minutes at the same temperature and decanted. The procedure of dilution, stirring and decantation was repeated two more times. The resulting red slurry was dissolved in methanol (25 mL), and trifluoroacetic acid (1 mL) was added dropwise at 5 °C. The solution was filtered and the filtrate was concentrated. The residue was purified by crystallization from diethyl ether (50 mL). The amorphous solid was washed with diethyl ether (2 × 50 mL) and dried under reduced pressure to yield trifluoroacetate 2 (1.00 g, HPLC purity = 80%, 1.15 mmol, 65.2%) as a red solid.

NMR spectra was measured from the free base.
¹H NMR (CDCl₃, 500 MHz, δ, ppm): 13.77 (1H, br s), 13.07 (1H, br s), 7.89 (d, 1H), 7.69 (t, 1H), 7.31 (d, 1H), 5.45 (t, 1H), 5.21 (br m, 1H), 5.12 (br d, 1H), 4.75 (s, 1H), 4.06 (qd, 1H), 4.01 (s, 3H), 3.85 (dd, 1H), 3.29 (q, 1H), 3.10-3.05 (m, 2H), 2.78(d, 1H), 2.58 (br q, 1H), 2.44-2.38 (m, 1H), 2.38 (s, 3H), 2.08-2.01 (m, 2H), 1.89-1.78 (m, 3H), 1.73-1.68 (m, 2H), 1.34 (d, 3H)
¹³C NMR (CDCl₃, 125 MHz, δ, ppm): 211.9, 186.5, 186.3, 160.8, 156.3, 155.6, 135.4, 135.2, 134.4, 134.1, 120.7, 119.6, 118.3, 111.1, 111.0, 100.7, 97.2, 76.8, 74.1, 69.3, 65.4, 58.8, 56.5, 54.3, 34.8, 33.1, 32.1, 31.1, 24.7, 24.2, 17.3

### Synthesis of 2-pyrrolinodaunorubicin (PyDau)-loaded sterically stabilized liposomes

Hydrogenated soy phosphocholine (HSPC, Coatsome NC-21E), 1,2-distearoyl-sn-glycero-3-phosphoethanolamine-N-[methoxy(polyethylene glycol)-2000] (DSPE-PEG2k, 880120P) were purchased from NOF Corporation (Japan) and from Avanti Polar Lipids (USA), respectively. Cholesterol (C8667), ammonium sulfate (A4418), L-Histidine (53319), sucrose (1.07687) and phosphate buffered saline (PBS, P3813) were purchased from Sigma-Aldrich (Hungary). All chemicals were used without further purification. The liposome sample was prepared by the hydration, freeze-thaw and extrusion method. The components were dissolved in chloroform in weight ratio of HSPC:DSPE-PEG2k:Cholesterol = 3:1:1. The solvent was then evaporated at 40 °C and the resulting lipid film was kept in vacuum overnight to remove residual traces of the solvent. 0.25 M ammonium sulfate (pH=6.5) solution made with ultrapure water (18.2 MQcm) was added to the sample to gain a total lipid concentration of 16 mg/ml. Ten freeze-thaw cycles by using liquid nitrogen and lukewarm water bath were applied for homogenization. Next, the samples were extruded (at 60°C) ten times through polycarbonate filters with 100 nm pore size (Nuclepore, Whatman Inc.) using a LIPEX extruder (Northern Lipids Inc., Canada). After the extrusion process, the external solution of the liposome sample was replaced by histidine-sucrose solution (10 mM/10 wt%, pH=6.5) using a PD-10 (GE Healthcare Life Sciences, USA) desalting column according to the manufacturer's instructions. Next, 1.1 mL of 2 mg/mL PyDau in 150 mM saline solution was added to 1.5 mL liposome solution and stirred at 60 °C for 1 hour. Finally, the PyDau loaded liposome sample was purified on a PD-10 column in two consecutive cycles using histidine-sucrose solution (10 mM/10 wt%, pH=6.5) as eluent.

### Characterization of PyDau liposomes

### Freeze- fracture combined transmission electron microscopy (FF-TEM)

FF-TEM enables the morphological characterization of soft materials in the aqueous phase by transmission electron microscopy (TEM). First, the sample is rapidly frozen and fractured in a vacuum chamber. The fractured surface is covered with platinum and carbon evaporation, which form a replica of the surface. This replica is then cleaned and transferred onto a copper grid and examined in a TEM instrument.

Samples were mixed with glycerol (G5516, Sigma-Aldrich, Hungary) used as cryoprotectant at 3:1 sample-to-glycerol volume ratio. Approx. 2 µL sample was frozen in Freon cooled with liquid nitrogen for 20 seconds. Fracturing was performed at -100 °C in a Balzers freeze-fracture device (Balzers BAF 400D, Balzers AG, Liechtenstein). The replicas of the fractured surfaces were made by platinum-carbon evaporation and then cleaned with a water solution of surfactant and washed with distilled water. The platinum-carbon replicas were placed on 200 mesh copper grids and examined in a MORGAGNI 268D (FEI, The Netherlands) transmission electron microscope.

### Dynamic light scattering (DLS)

DLS measurements were performed using a W130i apparatus (Avid Nano Ltd., UK) and using a low volume disposable cuvette (UVette, Eppendorf Austria GmbH, Austria).

### Microfluidic resistive pulse sensing (MRPS)

MRPS measurements were performed with an nCS1 instrument (Spectradyne LLC, USA). The final liposome sample was diluted 1000-fold with bovine serum albumin (BSA, A2153, Sigma-Aldrich, Hungary) solution at 1 mg/mL in PBS buffer, filtered through an Amicon Ultra-0.5 mL, 100 kDa MWCO membrane filter (Sigma-Aldrich, Hungary) according to the manufacturer's instructions. The sample was measured with factory calibrated TS-400 cartridges (65 nm to 400 nm measurement range).

Figure 12 shows a typical FF-TEM image of PyDau Liposomes. Monodisperse, spherical particles with an average diameter of 100 nm can be observed on the image. The hydrodynamic diameter of the PyDau Liposomes was 121.5±1.9 nm with polydispersity index of 10.6±2.5 % as determined by DLS. The size distribution of the liposomes determined by MRPS is shown in Figure 12b. The measured distribution can be fitted with a Gaussian function (R2 = 0.997) with 94.6 nm mean value and 16.5 nm standard deviation. FF-TEM, DLS and MRPS verify that the prepared liposomal system is homogeneous and has a narrow size distribution. The concentration of liposomes in the final product according to the MRPS measurement is 9.91*10¹³ 1/mL. The difference between the hydrodynamic diameter and the diameter determined by MRPS can be attributed to the thickness of the hydration layer of the liposomes. LC-MS was used to quantify the encapsulated drug in the liposomal formulation.

The concentration of PyDau in the final formulation was found to be 0.34±0.03 mg/mL, which corresponds to 77% loading efficiency considering the initial concentration of the drug used during the preparation.

Fig. 12C shows FF-TEM images of liposomal PyDox: aggregated liposomes with irregular surface patterns are visible on the images, while Fig. 12A shows FF-TEM images of liposomal PyDau: intact, spherical, non-aggregated liposomes are visible on the images.

### Small-angle X-ray scattering (SAXS)

SAXS is a measurement method for the structural characterization of materials at the nanoscale. In SAXS, a collimated and monochromatic X-ray beam is scattered on the electrons of the sample, and the photons scattered at low angles are collected with a position-sensitive X-ray detector. The primary result of the measurement is the so-called scattering curve, expressing the angular dependence of the intensity of the scattered radiation. The mathematical groundwork of scattering theory shows that the scattered intensity is the absolute square of the scattered amplitude (itself a complex value), which is in turn the Fourier transform of the excess electron density defined above. The variable conjugated to the real-space coordinate vector by virtue of the Fourier transform is termed the momentum transfer or scattering variable and is commonly denoted by q. Simple geometrical considerations lead to the relation of the magnitude of this vector to the scattered angle, following the formula q=4πsin ((θ))/λ, where 2θ is the scattering angle and λ is the wavelength of the incident (monochromatic) beam. This experimental method is a good choice for characterizing a broad class of various nanoscale systems, including nanoparticles, proteins, phospholipid membranes, other self-assembling systems etc. Here we employ it to assess the structure of doxorubicin and its analogues embedded in a sterically stabilized phospholipid vesicle.

### Materials and Methods

Scattering measurements of pyrrolino-doxorubicin (PyrDox) and pyrrolino-daunorubicin (PyrDau), as well as a reference sample of Caelyx (a commercial liposomal doxorubicin drug) were carried out in CREDO, an in-house built SAXS pinhole camera. Monochromatic Cu Kα X-rays (λ=0.154 nm) are generated using a GeniX3D Cu ULD integrated beam delivery system (produced by Xenocs SA, Sassenage, France), which features a 30 W microfocus X-ray tube, coupled to a parabolic graded multilayer Si/W mirror, which parallelizes the beam. The X-ray beam requires further shaping with a 3-pinhole collimation scheme to achieve the required beam size and divergence. After concentrating with ultracentrifugation, samples were put in thin-walled (~ 0.01 mm) borosilicate capillaries of approx. 1.3 mm outer radius, sealed with hot glue to withstand the vacuum of the evacuated sample chamber. Photons scattered by the sample are collected by a Pilatus-300k CMOS hybrid pixel detector (Dectris Ltd, Baden, Switzerland), placed 690.7 mm downstream from the sample. The above-described setup corresponds to a range of 0.143 nm⁻¹ < q < 4 nm⁻¹. Measurements were made repetitively in 5-minute parts, with frequent remeasuring of instrumental and external background noise and the calibration samples. Each exposure was corrected and calibrated using the standard online data reduction routine implemented in the instrument control software. Background signals, angle-dependent X-ray absorption of the sample and geometrical effects (detector flatness) were accounted for. The intensity was scaled into absolute units of differential scattering cross-section (with cm⁻¹ × sr⁻¹ dimension) using a piece of glassy carbon calibrated previously on the same instrument to the scattering of distilled water. The horizontal (q) axis (or conversely, the sample-to-detector distance) was calibrated using a mixture of silver behenate and SBA-15 mesoporous silica, also pre-calibrated on the same instrument using first-principles methods of detector shifting.

The two-dimensional scattering patterns were azimuthally averaged, yielding radial scattering curves. Repeated experiments on the same sample were averaged, filtering out measurements affected by artifacts of excess external radiation (cosmic rays, natural background), using Tukey's interquartile range test on the averaged least-squares differences of scattering curves recorded from the same sample.

### Results

Corrected, calibrated and averaged scattering curves of the three samples are shown in Fig. 14.

The monotonic decrease of the scattering curves is characteristic of particulate systems: in this case the liposomes and the encapsulated drug. The deflection at the beginning of the Caelyx reference system corresponds to the outer size of the liposomes and the encapsulated drug. In the same range, the liposomal PyDau and PyDox samples do not show the deflection, corresponding to either a larger size of the vesicles or the weaker signal of the drug aggregate, due to it being less ordered. The mid-range of the curves (from cca. 0.4 to 1.5 nm⁻¹) characterizes the orderliness of the phospholipid envelope through the so-called bilayer form factor. In Caelyx this overlaps with the scattering of the well-ordered doxorubicin aggregate. In the liposomal PyDau sample, the bilayer form factor is more pronounced than in the liposomal PyDox sample, pointing to a better-ordered layer in the former.

The peak around 2.2 nm⁻¹ originates from the doxorubicin liquid crystals inside the liposomes. Fitting a Lorentzian peak function using orthogonal distance regression to the scattering curve in the range of 1.6 < q < 4 nm⁻¹ yields a peak position of 2.273 ± 0.007 nm. With the help of sulfate anions, doxorubicin self-assembles in a "stack of coins" manner. These stacks in turn align in a nearly parallel fashion, ordered hexagonally. The peak position corresponds to the hexagonal order, with the lattice parameter of ~2.39 nm.

This peak is not found in the other two scattering curves, confirming that PyDox and PyDau do not form ordered structures inside the liposomes.

Conclusions
1. In contrast to liposomal doxorubicin, the state of the encapsulated drug in liposomal PyDau and PyDox is less ordered, the liquid crystalline phase of the encapsulated drug characteristic of liposomal doxorubicin is not observable for liposomal PyDau and PyDox as revealed by SAXS.
2. The phospholipid bilayer structure is less ordered for liposomal PyDox than for liposomal PyDau as revealed by SAXS.
3. FF-TEM images of liposomal PyDau shows intact, spherical, and non-aggregated particles, while liposomal PyDox contains aggregated liposomes with irregular surface patterns in line with the observation from SAXS discussed above.

### In vitro cytotoxicity studies

Cells were seeded in 40 µl medium on 384 well plates by an automatic liquid handling machine (Hamilton StarLet) in a density of 2500 cells/well. Adherent cells were cultured for 24 h before the addition of the compounds, while drugs were added to non-adherent cells 2 h after cell seeding. The drugs were serially diluted, and dispensed in an additional 20 µl. Cells were incubated for an additional 72 h, cytotoxicity was assessed by the PrestoBlue viability reagent (Thermo Fisher). Experiments were repeated at least 3 times.

Values for DOX and DAU against the matching NCI-60 cell lines are taken from the NCI DTP's NCI-60 cell line screen database as NSC123127 and NSC756717, respectively, measured by sulforhodamine B cell viability assay. Values for PANC-1 for DOX and DAU were taken from Varbanov et al. (2017) Repositioning approved drugs for the treatment of problematic cancers using a screening approach. PLoS ONE 12(2): e0171052., measured by PrestoBlue reagent on 384 well plates.

Human cancer cell lines shown on Figure 1:
melanoma (Malme-3M, SK-MEL-2, SK-MEL-28);
colorectal (COLO205, DLD-1, HCC-2998, HCT-116, HCT-15, HT29, KM12, SW-620);
lung (EKVX, NCI-H522, NCI-H1792, NCI-H322M, LCLC, HOP-62, NCI-H226);
prostate (PC-3, DU-145)
leukaemia (HL60, CCRF-CEM)
ovarian (OVCAR-3, OVCAR-5, OVCAR-8, NCI/ADR-RES);
renal (UO-31, A498, CAKI-1, RXF393)
breast cancers (BT-549, Hs578T, MDA-MB-231, MCF-7, T47D, MDA-MB-435).
pancreatic (PANC-1, Bx-PC-3).

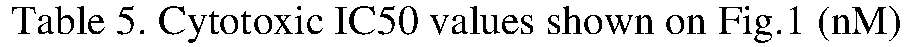

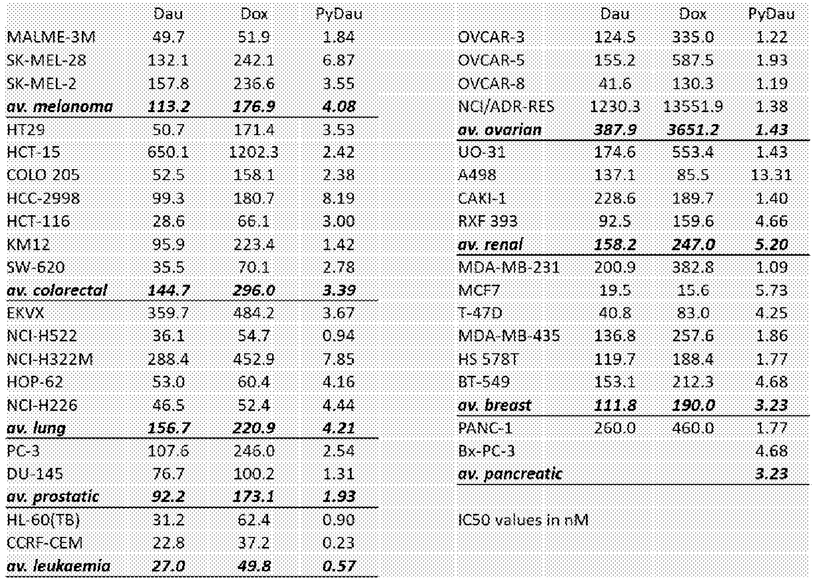

### In vivo toxicity studies

PyDau and LiPyDau dose-finding studies were done using 10 weeks old FVB mice, by single intravenous injections of different doses of the compounds (3 mouse/dose) via the tail vein. The status of the mice was monitored daily for signs of pain or discomfort. Mice were euthanized at 20% body weight loss or at a low score (1 or 2) on the Body Condition Scoring scale. Survival was followed for 30 days and the Maximum Tolerable Dose (MTD) was defined as the highest dose where 100% survival was observed.

PyDau proved to be lethal at 3, 2, 1 and 0.5 mg/kg with an MTD of 0.25 mg/kg, however later experiments suggested that this dose is still toxic when given repeatedly. In contrast, the MTD of LiPyDau was 1.5 mg/kg while 3 and 2 mg/kg doses were lethal.

### Antitumor activity of LiPyDau in mouse models

### Xenograft modeld

### Cell-line derived tumors in mice

LiPyDau efficacy was compared to doxorubicin in drug-naïve and drug-resistant xenograft tumors established from the uterine sarcoma cell lines MES-SA and its multidrug resistant derivative MES-SA/Dx5. 2×10⁶ cells from each cell line in 200 µl serum-free culture media DMEM were inoculated into NOD.Cg-*Prkdc^{scid} Il2rg^{tm1Wj}*/SzJ (NSG) mice subcutaneously in the left flank and treatment was initiated when tumors reached 200mm³. Mice were treated with saline (n=3 per each cell lines), 2 mg/kg doxorubicin (n=5 per each cell lines) or 0.5 mg/kg LiPyDau (n=5 per each cell lines) intravenously once per week for 3 times or until the tumor volume reached 2000mm³. The size of the tumors was measured every second day with a digital caliper.

### Human patient-derived tumor in mice

The 83-year-old patient was diagnosed with invasive lung adenocarcinoma in 2018 by the 2^{nd} Department of Pathology, Semmelweis University. The tumor was removed and sequencing revealed the lack of EGFR, KRAS or ALK mutations rendering the patient ineligible for available targeted therapy. A piece from the tumor tissue was transplanted subcutaneously into the right flank and propagated in NOD.Cg-*Prkdc^{scid} Il2rg^{tm1Wjl}*/SzJ (NSG) mice for 7 months. After the xenografted tumor reached 1000mm³, tumor pieces were retransplanted subcutaneously into the right flank of 20 additional NSG mice, which were given a single treatment with various chemotherapeutics or LiPyDau. Every treatment group consists of 5 animals. After daily monitoring of the tumor volume for one week, the mice were sacrificed and the cytotoxicity of the treatments was investigated on tissue slices by image analysis.

For treatment, tumor pieces were engrafted subcutaneously. To avoid biases at the start of treatment, tumor-bearing mice with similar tumor volume, tumor growth rate, and mouse weight were distributed evenly between the control (saline), doxorubicin (DOX, 2 mg/kg), paclitaxel (PCT, 12 mg/kg) or LiPyDau-treated groups. In contrast to DOX and PCT, which failed to exert any effect on tumor growth, LiPyDau profoundly suppressed the growth of patient-derived xenograft adenocarcinoma tumors (Figure 3).

### Mouse allograft models

Mouse melanoma (B16), lung (Lewis lung carcinoma, LLC) and breast (4T1-LUC) cancer cell lines were used to establish allograft tumors for drug testing. 5×10⁵ B16 or 2×10⁵ LLC cells were inoculated subcutaneously in 200 µl serum-free culture media. 4T1-LUC tumors were established by injecting 1×10⁶ cells in 20 µl serum-free culture media through the nipple directly to the breast tissue of 8 weeks old BALB/c mice on day 0. Efficacy of LiPyDau was compared to the first-line chemotherapy drugs used in the treatment of the given cancer type: dacarbazine for melanoma (n=6), cisplatin for lung cancer (n=7) and doxorubicin for breast cancer (n=6). 4T1-LUC tumor-bearing mice were randomly divided into three experimental groups receiving treatment by saline, doxorubicin (DOX), or LiPyDau on day 12 and 24. For the B16, LLC and 4T1-LUC tumor models 4, 6 and 6 mice were treated with LiPyDau while 4, 3 and 6 animals were in the saline treated groups, respectively. Growth of 4T1-LUC tumor was monitored by bioluminescence imaging. Images were taken 10 minutes after tail vein injection of 150 mg/kg dose of D-luciferin into the mouse.

### Genetically engineered mouse model of triple-negative breast cancer

The therapeutic value of LiPyDau was also tested in a clinically relevant murine model of hereditary triple negative breast cancer. Wild-type FVB mice were orthotopically transplanted with mammary tumor pieces obtained from Brca1^{-/-};p53^{-/-} mice. This model closely mimics cancer in human patients and therefore offers a unique opportunity to study the response to treatment. Like most human cancers, Brca1^{-/-};p53⁻¹⁻ tumors show initial sensitivity to doxorubicin and combination treatments such as FEC (5-fluorouracil, epirubicin and cyclophosphamide), but the tumors always acquire resistance (Figure 7). Saline-treated animals had to be sacrificed by day 12 because the tumor volume reached the limit of ~2000mm³. In some tumors, treatment with DOX resulted in an initial response, but all the DOX-treated tumors relapsed and became resistant to therapy within 60 days. While combination treatment with Fluorouracil-Epirubucin-Cyclophosphamide (FEC, 24, 4 and 120 mg/kg, respectively) further increased the life span of mice, eventually all mice had to be sacrificed because the tumors became refractory to treatment.

In the same model, treatment with LiPyDau resulted in a marked, dose-dependent antitumor response, efficiently curing several mice (Figure 8). At 0.5 mg/kg, all tumors showed a significant size reduction, leading to a complete response that however was only transient, although the recurring tumors remained sensitive to LiPyDau treatment. At 1 mg/kg, LiPyDau was more effective, resulting in almost 60 days of tumor-free survival (note that in 60 days tumors become refractory to DOX treatment). Finally, at 1.5 mg/kg, a single treatment with LiPyDau eradicated half of the tumors, with mice reaching more than 2 years of tumor-free survival. 3 mice within that group had to be sacrificed due to the toxic side effects of the treatment. To reduce the toxicity of LiPyDau, the 1.5 mg/kg dose was distributed to three 0.5 mg/kg doses, administered over 21 days (0.5 mg/kg/week). In that setting, 4 of the 5 mice were effectively cured, while one tumor relapsed after 80 days.

### Comparison of the antitumor activity of DOX, PLD, PYDau and LiPyDau in the genetically engineered mouse model of triple-negative breast cancer

Tissue pieces (1-2 mm in diameter) obtained from Brca1^{-/-};p53⁻¹⁻ FVB mouse mammary tumors (a kind gift from Sven Rottenberg, NKI) were transplanted orthotopically into the mammary fat pad of wild type FVB mice (Harlan) under anesthesia (20 mg/kg zolazepam, 12.5 mg/kg xylazine, 3 mg/kg butorphanol, 20 mg/kg tiletamine). The tumor size was monitored at least 3 times per week by caliper measurements after the tumors became palpable. Tumor volume was calculated using the V = length× (width²/2) formula. When the volume of the tumors reached ~200 mm³, DOX and PLD treatment was initiated using the maximum tolerable dose (MTD, 5 and 8 mg/kg iv. respectively). Treatments using the MTD were repeated every 10 days unless the size of the tumors decreased to 50% of its original volume. In that case treatment was repeated when the tumor relapsed to its original size. Animals were sacrificed when the tumor volume reached ~2000 mm³ (Fig. 13).

## Claims

1. Liposome-encapsulated 2-deamino-2-pyrrolino-daunorubicin (LiPyDau), for use in therapy.

2. The LiPyDau for use according to claim 1, wherein the therapy is a method for treating cancer.

3. The LiPyDau for use according to claim 2, wherein the cancer is selected from the group consisting of breast cancer, uterine cancer, lung cancer, melanoma, Kaposi's sarcoma, and neuroblastoma.

4. The LiPyDau for use according to claim 2 or 3, wherein the cancer is recurrent cancer.

5. The LiPyDau for use according to any one of claims 2 to 4, wherein the cancer is a therapy-resistant cancer.

6. The LiPyDau for use according to any one of claims 2 to 5, wherein the cancer has been previously treated with an anthracycline compound.

7. The LiPyDau for use according to claim 6, wherein the anthracycline compound is a non-liposomal doxorubicin and/or liposomal doxorubicin.

8. The LiPyDau for use according to any one of claims 2 to 7, wherein the cancer is a P-glycoprotein (Pgp) overexpressing cancer.

9. The LiPyDau for use according to any one of the preceding claims, wherein the liposome comprises:
- cholesterol, hydrogenated soy phosphatidylcholine (HSPC), 1,2-distearoyl-sn-glycero-3-phosphoethanolamine sodium salt (DSPE);
- cholesterol, HSPC, N-(carbonyl-methoxypolyethylene glycol 2000)-1,2-distearoyl-sn-glycero-3-phosphoethanolamine sodium salt (MPEG-DSPE);
- cholesterol, phosphatidylcholine, citric acid, NaOH or
- cholesterol and distearoylphosphatidylcholine.

10. Pharmaceutical composition comprising liposome-encapsulated 2-deamino-2-pirrolinodaunorubicin and a pharmaceutically acceptable excipient.

## Patentansprüche

1. Liposomen verkapseltes 2-Desamino-2-Pyrrolino-Daunorubicin (LiPyDau) zur Verwendung in der Therapie.

2. Das LiPyDau zur Verwendung gemäß Anspruch 1, wobei die Therapie ein Verfahren zur Behandlung von Krebs ist.

3. Das LiPyDau zur Verwendung gemäß Anspruch 2, wobei der Krebs ausgewählt ist aus der Gruppe bestehend aus Brustkrebs, Gebärmutterkrebs, Lungenkrebs, Melanom, Kaposi-Sarkom und Neuroblastom.

4. Das LiPyDau zur Verwendung gemäß Anspruch 2 oder 3, wobei der Krebs rezidivierender Krebs ist.

5. Das LiPyDau zur Verwendung gemäß einem der Ansprüche 2 bis 4, wobei der Krebs ein therapieresistenter Krebs ist.

6. Das LiPyDau zur Verwendung gemäß einem der Ansprüche 2 bis 5, wobei der Krebs zuvor mit einer Anthrazyklinverbindung behandelt wurde.

7. Das LiPyDau zur Verwendung gemäß Anspruch 6, wobei die Anthrazyklinverbindung ein nicht-liposomales Doxorubicin und/oder liposomales Doxorubicin ist.

8. Das LiPyDau zur Verwendung gemäß einem der Ansprüche 2 bis 7, wobei der Krebs ein P-Glykoprotein (Pgp) überexprimierender Krebs ist.

9. Das LiPyDau zur Verwendung gemäß einem der vorhergehenden Ansprüche, wobei das Liposom
- Cholesterin, hydriertes Sojaphosphatidylcholin (HSPC), 1,2-Distearoyl-sn-Glycero-3-Phosphoethanolamin-Natriumsalz (DSPE);
- Cholesterin, HSPC, N-(Carbonylmethoxypolyethylenglykol 2000)-1,2-Distearoyl-sn-Glycero-3-Phosphoethanolamin-Natriumsalz (MPEG-DSPE);
- Cholesterin, Phosphatidylcholin, Zitronensäure, NaOH oder
- Cholesterin und Distearoylphosphatidylcholin
umfasst.

10. Pharmazeutische Zusammensetzung, die liposomen verkapseltes 2-Desamino-2-Pyrrolino-Daunorubicin und einen pharmazeutisch verträglichen Hilfsstoff enthält.

## Revendications

1. 2-Déamino-2-pyrrolino-daunorubicine encapsulée dans des liposomes (LiPyDau), pour son utilisation en thérapie.

2. La LiPyDau pour son utilisation selon la revendication 1, dans lequel la thérapie est une méthode de traitement du cancer.

3. La LiPyDau pour son utilisation selon la revendication 2, dans lequel le cancer est choisi dans le groupe constitué par le cancer du sein, le cancer de l'utérus, le cancer du poumon, le mélanome, le sarcome de Kaposi et le neuroblastome.

4. La LiPyDau pour son utilisation selon la revendication 2 ou 3, dans lequel le cancer est un cancer récurrent.

5. La LiPyDau pour son utilisation selon l'une quelconque des revendications 2 à 4, dans lequel le cancer est un cancer résistant à la thérapie.

6. La LiPyDau pour son utilisation selon l'une quelconque des revendications 2 à 5, dans lequel le cancer a été préalablement traité avec un composé d'anthracycline.

7. La LiPyDau pour son utilisation selon la revendication 6, dans lequel le composé d'anthracycline est une doxorubicine non liposomale et/ou une doxorubicine liposomale.

8. La LiPyDau pour son utilisation selon l'une quelconque des revendications 2 à 7, dans lequel le cancer est un cancer surexprimant la glycoprotéine P (Pgp).

9. La LiPyDau pour son utilisation selon l'une quelconque des revendications précédentes, dans lequel le liposome comprend :
- cholestérol, phosphatidylcholine de soja hydrogénée (HSPC), sel de sodium de 1,2-distéaroyl-sn-glycéro-3-phosphoéthanolamine (DSPF) ;
- cholestérol, HSPC, sel sodique de N-(carbonyl-méthoxypolyéthylène glycol 2000)-1,2-distéaroyl-snglycéro-3-phosphoéthanolamine (MPEG-DSPE) ;
- cholestérol, phosphatidylcholine, acide citrique, NaOH ou
- cholestérol et distéaroylphosphatidylcholine.

10. Composition pharmaceutique comprenant de la 2-déamino-2-pirrolinodaunorubicine encapsulée dans des liposomes et un excipient pharmaceutiquement acceptable.
